# EUROPEAN PATENT APPLICATION

(11) **EP 4 765 144 A1**
(43) Date of publication of application: **24.06.2026**
(21) Application number: 25219091.3
(22) Date of filing: 27.11.2025
(51) Int. Cl.: G16H 20/60, A61B 5/00, G16H 50/70, G16H 50/50

(54) **SYSTEM AND METHOD FOR PROVIDING A RECOMMENDED HYDRATION METHOD**

(30) Priority: 17.12.2024 EP 24220501
(71) Applicant: Stichting IMEC Nederland, 6708 WH Wageningen (NL)
(72) Inventor: KHO, Esther, 6709 VK Wageningen (NL); GAITAN, Santiago, 2493 CH The Hague (NL); VAN KRAAIJ, Alex, 5361 HR Grave (NL); VAN STIPHOUT, Rudi, 6604 DN Wijchen (NL); THAMMASAN, Nattapong, 4105 XD Culemborg (NL)
(74) Representative: EIP

(57) **Abstract**

The present disclosure provides a system comprising one or more processors; and one or more input devices for providing data, wherein the system is configured to receive, from a first input device of the one or more input devices, hydration context data indicative of a hydration context of a user; estimate, using at least a first machine learning, ML, model and based on the hydration context data, a hydration level of the user; simulate, using the first ML model, one or more hydrating methods for hydrating the user; and determine, based on the one or more hydrating methods, a recommended hydration method for the user.

## Description

### Technical Field

The present invention relates to a system and a method for providing a recommended hydration method for a user.

### Background

Dehydration is a condition that occurs when a body loses more fluids than it takes in, leading to a deficiency of water and other fluids necessary for normal bodily functions. If dehydration remains untreated, it can lead to serious complications such as development of disorders like urinary tract infections, acute kidney injury and constipation, higher mortality rates, and significant medical costs.

Clinical symptoms and signs of dehydration, however, generally have poor sensitivity and specificity, and dehydration can develop very rapidly, which makes it difficult to diagnose.

Certain groups are more vulnerable to get dehydrated due to various factors such as age, health status, and environmental conditions. A particularly vulnerable group is elderly people due to their decrease of total body water, decreasing kidney function and decreased thirst stimulus. Even for healthy, younger people, staying hydrated can be beneficial in daily life, for example to avoid tiredness, concentration problems, headaches and mood disturbances.

In view of the above, there is a need for a method that can monitor and predict a future hydration status of a user and provide recommendations on how to prevent dehydration.

### Summary

It is an object of the present invention to provide a system and a method, which can predict of a future hydration status of a user and provide a recommended hydration method to the user. Further and alternative objectives may be understood from the following.

According to a first aspect of the present invention, there is provided a system comprising one or more processors and one or more input devices for providing data. The system is configured to receive, from a first input device of the one or more input devices, hydration context data indicative of a hydration context of a user. The first input device may be a urine sensor and the hydration context data may be a urine hydration level, such as a urine specific gravity (USG), of the user. The system is configured to estimate, using at least a first machine learning (ML) model and based on the hydration context data, a hydration level, such as a current and/or future hydration level, of the user. The system is configured to simulate, using the first ML model, one or more hydrating methods for hydrating the user. The system is further configured to determine, based on the one or more hydrating methods, a recommended hydration method for the user. The hydration method may be indicative of one or more of a type of liquid to consume and a time to consume the liquid.

As used herein, data refers to information indicative of one or more parameters, states, conditions, or characteristics of a system, process, or environment, in this case of a user's hydration state, which may be obtained, measured, calculated, or derived from sensors, user input, computational models, or other sources.

The system may provide customized hydration recommendations by considering individual hydration context of the user and simulating multiple hydration methods using machine learning. The integration of hydration context data may allow for more accurate assessment of user hydration, such as rehydration, needs compared to fixed hydration schedules. Simulating a plurality of hydration methods enables identification of the most effective approach for maintaining optimal hydration levels for each specific user

According to a second aspect of the present invention, there is provided a computer implemented method for providing a recommended hydration method for a user. The method comprises obtaining hydration context data indicative of a hydration context of a user. The method comprises estimating, using at least a first ML model and based on the hydration context data, a hydration level, such as a current and/or future hydration level, of the user. The method comprises simulating, using the first ML model, one or more hydrating methods for hydrating the user. The method comprises determining, based on the one or more simulated hydrating methods, a recommended hydration method for the user.

The method enables real-time determination and adaptation of hydration recommendations by combining hydration context data analysis with ML-based simulation of multiple hydration methods. The method enables real-time adaptation of hydration recommendations based on continuously updated context data and machine learning predictions. By simulating a plurality of hydration methods, the different hydration methods may be evaluated before being recommended to the user, thereby optimizing the hydration of the user and reducing the risk of dehydration. By integrating the ML model with context data, a selection of hydration methods tailored to individual user circumstances can be enabled.

Hydrating the user can herein be seen as keeping the user hydrated, such as upon the estimated hydration level of the user is above a hydration threshold, or rehydrating the user, such as upon the estimated hydration level of the user is equal to or below the hydration threshold. The hydration threshold may be a personalized threshold, such as a user specific threshold.

In one or more examples, the system is configured to predict, using the first ML model trained to predict a future hydration level based on the hydration context data, a future hydration level of the user.

The system may further be configured to estimate, using a second ML model trained to estimate a current hydration level based on the hydration context data, an estimated current hydration level of the user.

By combining a current and a future hydration level prediction/estimation a more complete understanding of the user's hydration status can be provided, which allows for an improved hydration method recommendation.

Using separate ML models for current and future hydration level estimation and/or prediction provides enhanced accuracy through specialized prediction capabilities and enables both a current hydration state and a future hydration state prediction which allows for a more informed hydration method recommendation for the user.

In one or more example methods, the first ML model and the second ML model may be the same ML model. In other words, the current hydration level of the user may be estimated using the first ML model trained to predict a future hydration level of the user by setting prediction time used as input to the first ML model to the current time.

In one or more examples, the system is configured to input a plurality of sets of hydration context data, wherein each of the sets of hydration context data is associated with a respective hydrating method, to the first ML model. The system may further be configured to simulate a respective predicted future hydration level for each set of the plurality of sets of hydration context data. Simulating multiple sets of hydration context data enables a quantitative comparison of potential outcomes for different hydration methods, which allows a selection of a recommended hydration method for the user.

In one or more examples, the system is configured to receive, from the one or more input devices, such as from the first input device or a second input device, ground truth data indicative of an actual hydration level of the user. The ground truth data may be received from a urine sensor. The ground truth data indicative of the actual hydration level of the user may be the urine hydration level, such as the USG, of the user

In one or more examples, the system is configured to compare the obtained ground truth data with ground truth data used to train the first ML model and/or the second ML model to determine whether the obtained ground truth data comprises ground truth data values not previously used for training the first ML model and/or the second ML model.

In one or more examples, the system is configured to determine that a number of ground truth data values not previously used for training the first and/or the second ML model is equal to or above a label threshold. The system may be configured to compare the received ground truth data values with the ground truth data used for training the first ML model, to determine whether there are ground truth data values available that have not yet been used to train the first and/or the second ML model.

In one or more examples, the system is configured to retrain, using the one or more processors, one or more of the first ML model and the second ML model based on the number of ground truth data values not previously used for training. The system may be configured to retrain the first and/or the second ML model upon determining that the number of ground truth data values not previously used for training the first and/or the second ML model is equal to or above the label threshold.

By retraining the first ML model and/or the second ML model automatically based on new ground truth data values not previously used for training the ML models ensures that the ML models maintain accuracy as user patterns may change over time.

In one or more example systems, the first input device is a sensor or a user interface. In one or more examples, the sensor is a hydration status sensor, such as a urine sensor. The urine sensor may be a sensor measuring the USG of the user. By configuring the system to support both sensor-based and user interface inputs, a broader application of the system across different usage scenarios and user preferences can be enabled. In one or more examples, such as when no urine sensor is available, the user may manually input information, such as raw data, indicative of the hydration level via the user interface. The manually input information, such as the raw data, indicative of the hydration level, may be used as input data to the first ML model and/or the second ML model in the absence of any ground truth data indicative of an actual hydration level of the user received from the urine sensor. In other words, in the embodiments described herein the ground truth data indicative of the hydration level of the user may be replaced with the manually input information, such as the raw data, indicative of the hydration level of the user.

In one or more examples, estimating the hydration level of the user comprises predicting, using the first ML model trained to predict a future hydration level based on the hydration context data, a future hydration level.

In one or more examples, estimating the hydration level of the user comprises estimating, using a second ML model trained to estimate a current hydration level based on the hydration context data, an estimated current hydration level.

In one or more example methods, the first ML model and the second ML model may be the same ML model. In other words, the current hydration level of the user may be estimated using the first ML model trained to predict a future hydration level of the user by setting prediction time used as input to the first ML model to the current time.

In one or more examples, simulating the one or more hydrating methods comprises inputting a plurality of sets of hydration context data, wherein each of the sets of hydration context data is associated with a respective hydrating method, to the first ML model. In one or more example methods, a first set of hydration context data may comprise a first type of liquid intake, such as a first type of drink, and a first intake time, such as a timestamp for the intake. In one or more example methods, a second set of hydration context data may comprise the first type of liquid intake, such as the first type of drink, and a second intake time. In one or more example methods, a third set of hydration context data may comprise a second type of liquid intake, such as a second type of drink, and the first intake time. In one or more example methods, a fourth set of hydration context data may comprise the second type of liquid intake, such as the second type of drink, and the second intake time. In one or more example methods, the sets of hydration context data may further comprise data indicative of a physical activity, a food intake, or any other hydration context data described herein.

In one or more examples, simulating the one or more hydrating methods comprises simulating a respective predicted future hydration level for each set of the plurality of sets of hydration context data. By simulating the one or more hydrating methods, simulated hydration values for different dietary intake scenarios (such as comprising a drink time and drink type) may be derived based on a combination of the hydration context data described herein. The simulated hydration methods may be compared to provide the user information on which dietary intake to consume and when to consume it to prevent dehydration.

In one or more examples, simulating the one or more hydration methods is performed upon the estimated hydration level being equal to or below a hydration threshold. Triggering the simulation only when the hydration levels fall below a threshold, reduces the computational resources for the simulation while maintaining effective hydration management.

In one or more examples, simulating the one or more hydration methods may be performed regardless of the estimated hydration level being above, equal to, or below the hydration threshold. Thereby the user can be provided with a recommended hydration method for maintaining a hydration level above the hydration threshold and reducing the risk of the hydration level reaching the hydration threshold.

In one or more examples, determining a recommended hydration method for the user comprises determining at least one of the plurality of sets of hydration context data resulting in a predicted future hydration level being above the hydration threshold. Selection of hydration methods based on a predicted achievement of the hydration threshold ensures effectiveness of the recommendation. The method ensures that recommended hydration methods will achieve adequate hydration levels by validating the hydration method against defined thresholds.

In one or more examples, the method comprises outputting the determined recommended hydrating method to a user device. By outputting the recommendation directly to the user device an immediate access can be provided to the user to the hydration recommendation for timely implementation of the hydration recommendation.

The hydration context data can herein be seen as data indicative of circumstances that may influence the hydration level of the user. The hydration context data is used as input to the first ML model and/or the second ML model for determining a current or a predicted future hydration level and/or a corresponding hydration method for the user.

In one or more examples, obtaining hydration context data comprises one or more of receiving the hydration context data from a first sensor, and receiving the hydration context data from a user interface.

In one or more examples, the hydration context data comprises data indicative of a dietary intake. The data indicative of the dietary intake may comprise data indicative of one or more of a type of liquid, an amount of the liquid, a type of food, an amount of food, a nutrient content of the liquid, a nutrient content of the food, and a time of day of the intake of the food or liquid, taken in by the user. The data indicative of the dietary intake, such as the type of liquid and/or food, and/or the nutrient content of the liquid and/or food may be used to determine the effect of the food and/or liquid on the simulated hydration level for that food and/or liquid type.

In one or more examples, the hydration context data comprises data indicative of a physical activity of the user. The data indicative of the physical activity of the user, such as an intensity, a duration or a type of the physical activity, may for example be received from a physical activity sensor, using one or more monitoring sensors for monitoring a heart rate (variability) of the user, skin conductance, or an accelerometer-based movement.

In one or more example methods, the hydration context data comprises data indicative of one or more of a breathing rate, a heart rate, a blood pressure, bioimpedance, skin conductance data indicative of a sweating of the user. The breathing rate, the heart rate, the blood pressure and/or the skin conductance data may be indicative of a physical activity which may affect the hydration level of the user.

In one or more examples, the hydration context data comprises data indicative of a geographical location of the user. The data indicative of the geographical location may be indicative of location coordinates, such as one or more of a longitudinal coordinate, a latitudinal coordinate, and an altitude. The geographical location of the user may be indicative of a climate, such as a temperature, a season, a time of the year, a humidity or accessibility of water supply at the location of the user. In one or more examples, the geographical location of the user may be received via a sensor, such as a global positioning system (GPS) sensor, or as user input via the user interface.

In one or more examples, the hydration context data comprises data indicative of one or more temperatures associated with the user. The one or more temperatures associated with the user may be indicative of a body temperature of the user and/or an ambient temperature at the user. The data indicative of the one or more temperatures associated with the user may be received by one or more temperature sensors.

In one or more examples, the hydration context data comprises data indicative of a dietary intake preference of the user. The data indicative of the dietary intake preference of the user may be received as user input via the user interface. The data indicative of the dietary intake preference of the user may be used to determine a recommended hydration method tailored to the user, for example by providing a personalised dietary intake recommendation.

In one or more examples, the hydration context data comprises data indicative of a health status, such as historical and current (chronic) disease states, physical complaints, fitness level, and mental well-being. The data indicative of the health status may be received as user input via the user interface.

In one or more examples, the hydration context data comprises data indicative of a demographic of the user, such as an age, a gender, or a body-mass index (BMI) of the user. The data indicative of the demographic of the user may be received as user input via the user interface.

In other words, simulating the one or more hydrating methods for the user, may be based on hydration context data indicative of one or more of the physical activity of the user, the geographical location of the user, the temperature associated with the user, the dietary intake preference of the user, the health status of the user, and the demographic of the user.

The hydration context data may comprise a corresponding time stamp for when the hydration context data was determined, such as measured by the sensor or input using the user interface. The time stamp allows a prediction of the hydration level of the user based on a time between the estimation and/or prediction of the hydration level and the time stamp of the hydration context data.

Incorporation of multiple contextual factors, such as multiple hydration context data, enables more nuanced and accurate hydration recommendations that account for diverse user circumstances.

In one or more examples, the first ML model is trained based on hydration context data and ground truth data indicative of an actual hydration level of the user, to output a future hydration level of the user. The hydration context data comprises at least data indicative of a dietary intake of the user. The ground truth data may be measured hydration level data, such as measured urine hydration level data, of the user. The measured urine hydration level data may be indicative of a USG of the user. The USG is a measure of the concentration of solutes (such as salts, waste products, and other substances) in the urine compared to the density of water. The USG may reflect the kidney's ability to concentrate or dilute urine based on the body's hydration and metabolic needs. Training the first ML model with dietary intake data enables more accurate prediction of future hydration levels by accounting for nutritional impacts on the hydration status of the user.

The recommended hydration method may be indicative of one or more actions to be taken by the user to restore or maintain their hydration level. The one or more hydration methods may be indicative of one or more of a type of dietary intake, such as a type of liquid or food to consume, and a time for the dietary intake, such as a time to consume the liquid or food. In one or more example methods, the recommended hydration method may be indicative of a change in physical activity and/or geographical location of the user.

The first input device may be a sensor or a user interface. Supporting both sensor-based and manual input methods via the user interface ensures broader applicability across different user scenarios and device configurations. In one or more examples, the sensor is configured to measure a hydration status of the user. The sensor may thus be a hydration status sensor, such as a urine sensor. The urine sensor may be a sensor attached to a toilet seat or integrated in a sanitary product, such as a diaper, for measuring the urine hydration level of the user during a toilet visit. Thereby, a hydration status of the user may be measured in a continuous, such as every time a user goes to the toilet, non-invasive and accurate manner without the need for any manual action.

In one or more examples, the sensor may be configured to determine a urine temperature to be used as a proximation of a body temperature of the user.

In one or more examples, the sensor may be configured to determine a weight loss and/or a weight increase of the user. The sensor may for example be a load cell arranged under the toilet seat.

In one or more examples, the sensor may be a sensor to track a lifestyle and context of the user, such as a dietary intake of the user, such as a type of liquid consumed, a type of food consumed, a quantity consumed per type of liquid, a quantity consumed per type of food, a time of day of the intake of the food and/or liquid, an ambient temperature of the user, a physical activity of the user, a fluid loss of the user.

In one or more examples, the user interface may be configured to receive hydration context data from the user, such as data indicative of personal preferences of the user, such as food and/or liquid preferences of the user, a nutrient content of the food and/or liquid, and a time of day of the intake of the food and/or liquid, taken in by the user.

In one or more example methods, the second ML model is trained based on hydration context data and ground truth data indicative of an actual hydration level of the user to output an estimated current hydration level of the user. The ground truth data indicative of an actual hydration level of the user may be urine hydration level data received from a urine sensor. The hydration context data may comprise data indictive of one or more of a dietary intake of the user and a urine hydration level of the user. The urine hydration level may be received from the urine sensor. Integration of both dietary intake and urine hydration data in the second ML model provides more comprehensive and accurate estimation of current hydration status

In one or more examples, the method comprises obtaining ground truth data indicative of an actual hydration level of the user. The ground truth data may for example be data indicative of a urine hydration level of the user, such as data received from the urine sensor.

In one or more examples, the method comprises comparing the obtained ground truth data with ground truth data used to train the first ML model and/or the second ML model to determine whether the obtained ground truth data comprises ground truth data values not previously used for training the first ML model and/or the second ML model. In one or more examples, the method comprises, upon determining that a number of ground truth data values not previously used for training the first and/or the second ML model is equal to or above a label threshold, retraining one or more of the first ML model and the second ML model based on the ground truth data values not previously used for training. By retraining the first ML model and/or the second ML model upon the number of ground truth data values not previously used for training the ML model being equal to or above a label threshold allows the ML models to maintain accuracy and adapt to changes in the user patterns over time, while reducing the computational resources required and the time the ML models are unavailable for retraining.

Further features and advantages of the invention will become apparent from the following description of preferred embodiments of the invention, given by way of example only, which is made with reference to the accompanying drawings.

### Brief Description of the Drawings

Figure 1 shows a block diagram illustrating an example system according to the present disclosure,
Figure 2 shows a flow diagram of an example method for providing a recommended hydration method according to the present disclosure,
Figure 3 is a graph illustrating an example comparison of different hydration method simulations according to the present disclosure, and
Figure 4 is a graph illustrating an example method for providing a recommended hydration method according to the present disclosure.

### Detailed Description

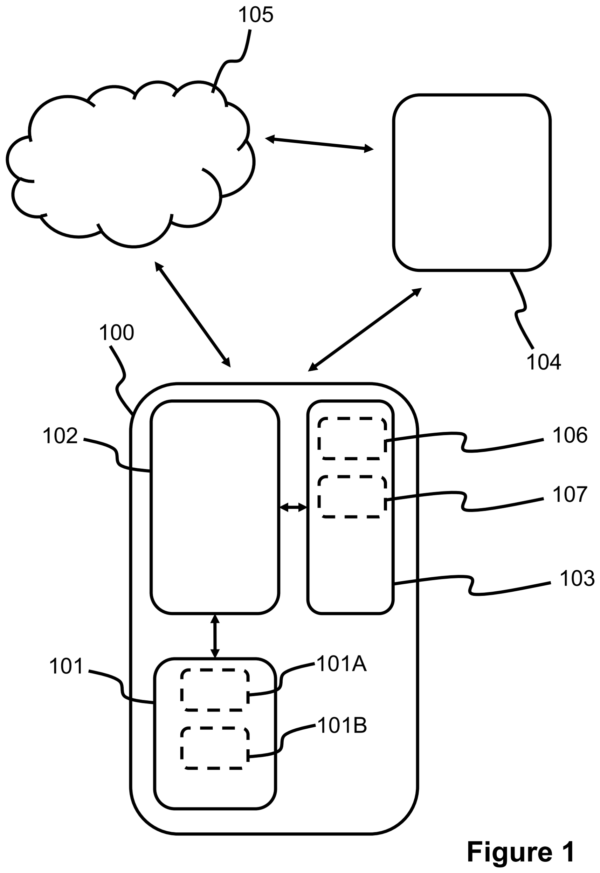

Figure 1 is a block diagram illustrating a system 100 according to the present disclosure, such as a system for determining a recommended hydration method for a user. The system 100 comprises one or more input device(s) 101 for providing data, one or more processor(s) 102, and memory 103 communicatively coupled with the one or more processor(s) 102. The one or more processors 102 are configured to receive data from the one or more input devices 101 and to execute instructions to process data and perform operations as described herein. The instructions may be instructions stored on the memory circuitry 103. The instructions stored on the memory circuitry 103 may comprise ML models, such as a first ML model 106 and/or a second ML model 107. The one or more processors 102 may be configured to communicate with one or more other devices, such as a user device 104, either directly or via a communications network 105.

The memory 103 may be an example of a non-transitory computer-readable media. The memory 103 may store an operating system and one or more software applications, instructions, programs, and/or data to implement the methods described herein and the functions attributed to the various systems. In various implementations, the memory may be implemented using any suitable memory technology, such as static random-access memory (SRAM), synchronous dynamic RAM (SDRAM), nonvolatile/Flash-type memory, or any other type of memory capable of storing information.

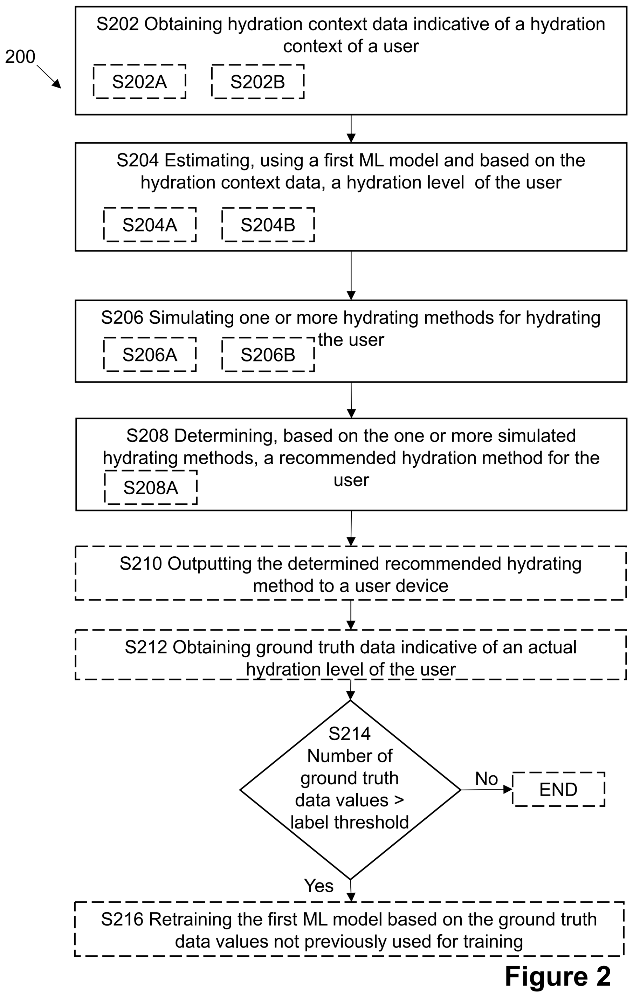

The system 100, such as the one or more processors 102, is/are configured to perform a method for providing a recommended hydration method for a user, such as the method 200 of Figure 2. In other words, the system 100, such as the one or more processors 102, is/are configured to receive from a first input device 101A of the one or more input device(s) 101, hydration context data indicative of a hydration context of a user. The system 100 is configured to estimate, using at least the first ML model 106 and based on the hydration context data, a hydration level of the user, such as a current hydration level or a predicted future hydration level of the user. In one or more examples, the one or more processor(s) 102 of the system 100 is/are configured to estimate, using at least the first ML model 106 and based on the hydration context data, the hydration level of the user. The system 100, such as the one or more processors 102, is/are is configured to simulate, using the first ML model, one or more hydrating methods for hydrating the user. The system 100, such as the one or more processors 102, is/are further configured to determine, based on the one or more hydrating methods, a recommended hydration method for the user. The first ML model may be stored on the memory circuitry 103.

In one or more examples, the system 100, such as the one or more processors 102, is/are configured to output the determined recommended hydrating method to the user device 104.

In one or more examples, the system 100, such as the one or more processors 102, is configured to predict, using the first ML model 106 trained to predict a future hydration level based on the hydration context data, a future hydration level. In one or more examples, the first ML model may be trained with urine sensor data (such as UGL data) and hydration context data as input and a predicted future hydration status of the user as output.

The system 100, such as the one or more processors 102, may further be configured to estimate, using a second ML model 107 trained to estimate a current hydration level based on the hydration context data, an estimated current hydration level. In one or more examples, the second ML model may be trained with urine sensor data (such as UGL data) and hydration context data as input and estimated current hydration status of the user as output.

In one or more example methods, the first ML model and/or the second ML model may be a machine learning algorithm. The machine learning algorithms may be based on one or more methods, such as Regression (Linear, Ridge, Lasso, Logistic, Elastic Net), Extreme Gradient Boosting (XGBoost), Random Forest, Support Vector Machines, or Neural Networks (Feedforward, Convolutional, Recurrent). This list is however not exhaustive and other machine learning models may also be used.

In one or more examples, the system 100, such as the one or more processors 102, is configured to input a plurality of sets of hydration context data, wherein each of the sets of hydration context data is associated with a respective hydrating method, to the first ML model. The system 100, such as the one or more processors 102, may further be configured to simulate a respective predicted future hydration level for each set of the plurality of sets of hydration context data.

In one or more examples, the system 100, such as the one or more processors 102, is configured to receive, from a second input device 101B, ground truth data indicative of an actual hydration level of the user.

In one or more examples, the system 100, such as the one or more processors 102, is configured to compare the obtained ground truth data with ground truth data used to train the first ML model 106 and/or the second ML model 107 to determine whether the obtained ground truth data comprises ground truth data values not previously used for training the first ML model 106 and/or the second ML model 107.

In one or more examples, the system 100, such as the one or more processors 102, is configured to determine that a number of ground truth data values not previously used for training the first ML model 106 and/or the second ML model 107 is equal to or above a label threshold.

In one or more examples, the system 100, such as the one or more processors 102, is configured to retrain, using the one or more processors, one or more of the first ML model 106 and the second ML model 107 based on the number of ground truth data values not previously used for training.

Figure 2 shows a flow diagram of an example method 200, such as a computer implemented method, for providing a recommended hydration method for a user. The method 200 comprises obtaining S202 hydration context data indicative of a hydration context of a user. In one or more example methods, obtaining S202 the hydration context data comprises receiving S202A the hydration context data from a first sensor. In one or more example methods, obtaining S202 the hydration context data comprises receiving S202B the hydration context data from a user interface.

The hydration context data may be used as input data to the first ML model and/or the second ML model to determine a recommended hydration method for the user. The hydration context data may comprise data indicative of one or more of a dietary intake, a physical activity of the user, a geographical location of the user, one or more temperatures associated with the user, such as a body temperature or an ambient temperature, a dietary intake preference of the user, a health status of the user, shower data of the user, a breathing rate of the user, a heart rate of the user, a blood pressure of the , skin conductance data indicative of a sweating of the user and a demographic of the user. The data indicative of the dietary intake comprises data indicative of one or more of a type of liquid, an amount of liquid, a nutrient content of the liquid, a type of food, an amount of food, a nutrient content of the food, and a time of day of the intake of the food and/or liquid, taken in by the user.

The method comprises estimating S204, using at least a first ML model and based on the hydration context data, a hydration level, such as a current and/or future hydration level, of the user. In one or more example methods, estimating S204 comprises predicting S204A, using the first ML model trained to predict a future hydration level based on the hydration context data, a future hydration level. The future hydration level may be a hydration level that the user is predicted to have at a future point in time.

In one or more example methods, estimating S204 comprises estimating S204B, using the first ML model and/or a second ML model trained to estimate a current hydration level based on the hydration context data, an estimated current hydration level. The current hydration level may be a hydration level that the user is estimated to have at a current point in time. In one or more example methods, the current hydration level of the user may be determined using the first ML model by setting the simulated time to the current time.

In one or more example methods, the first ML model is trained based on hydration context data and ground truth data indicative of an actual hydration level of the user to output a future hydration level of the user. The hydration context data may comprise at least data indictive of a dietary intake of the user. In one or more example methods, the hydration context data may comprise any of the hydration context data mentioned herein. The ground truth data may comprise a urine hydration level of the user, such as a measured urine hydration level of the user received from a urine sensor.

In one or more example methods, the second ML model is trained based on hydration context data and ground truth data indicative of an actual hydration level of the user to output an estimated current hydration level of the user. The hydration context data may comprise data indictive of one or more of a dietary intake of the user. The ground truth data may comprise a urine hydration level of the user, such as a measured urine hydration level of the user received from a urine sensor.

The method comprises simulating S206, using the first ML model, one or more hydrating methods for hydrating the user. In one or more example methods, simulating S206 the one or more hydrating methods comprises inputting S206A a plurality of sets of hydration context data, wherein each of the sets of hydration context data is associated with a respective hydrating method, to the first ML model. In one or more example methods, simulating S206 the one or more hydrating methods further comprises simulating S206B a respective predicted future hydration level for each set of the plurality of sets of hydration context data. In one or more example methods, simulating the one or more hydrating methods is performed upon the estimated hydration level being equal to or below a hydration threshold.

The method comprises determining S208, based on the one or more simulated hydrating methods, a recommended hydration method for the user. In one or more example methods, determining S208 comprises determining S208A at least one of the plurality of sets of hydration context data resulting in a predicted future hydration level being above the hydration threshold.

In one or more examples, the method comprises outputting S210 the determined recommended hydrating method to a user device. The one or more hydration methods may be indicative of one or more of a type of dietary intake, such as a type of liquid or food to consume, and a time for the dietary intake, such as a time to consume the liquid or food. In one or more examples, the recommended hydrating method may be indicative of a recommended change of physical activity or geographical position of the user.

In one or more examples, the method comprises obtaining S212 ground truth data indicative of an actual hydration level of the user, such as an USG of the user. The ground truth data may be received from the urine sensor.

In one or more examples, the method comprises comparing S214 the obtained ground truth data with ground truth data used to train the first ML model and/or the second ML model to determine whether the obtained ground truth data comprises ground truth data values not previously used for training the first ML model and/or the second ML model.

In one or more examples, the method comprises, upon determining that a number of ground truth data values not previously used for training the first and/or the second ML model is equal to or above a label threshold, retraining S216 one or more of the first ML model and the second ML model based on the ground truth data values not previously used for training.

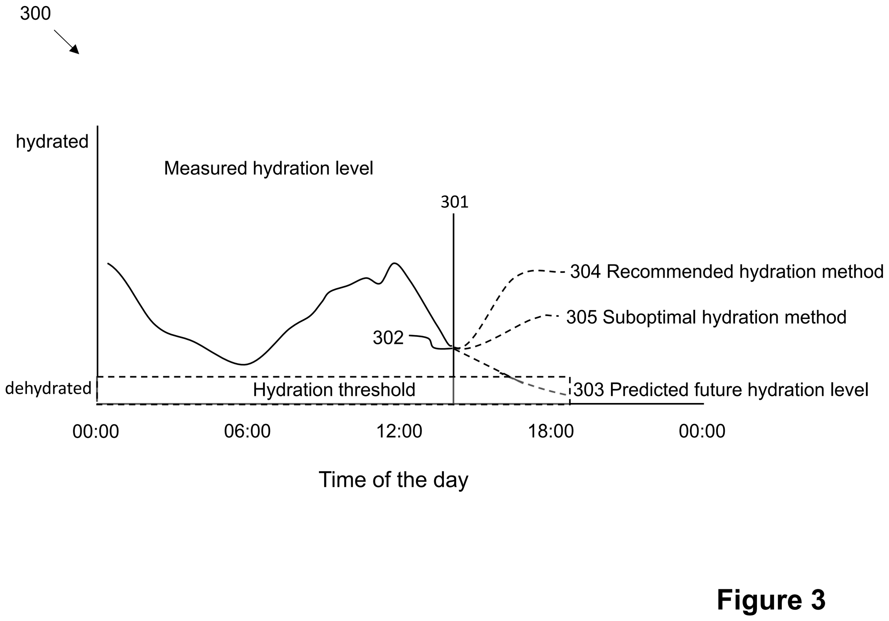

Figure 3 is a graph 300 illustrating a comparison of different hydration method simulations and determination of a recommended hydration method for the user according to one or more examples herein. The hydration level of the user may be monitored throughout a day, for example by receiving measured hydration levels, such as urine hydration levels of the user, at predetermined measurement instances. Based on the received measured hydration levels, an estimated current hydration level 302 and a predicted future hydration level 303 of the user may be determined at a current time 301. The predicted future level 303 may represent a hydration level expected to occur if no intervention is applied. If the estimated current hydration status or the predicted future hydration status is equal to or lower than the hydration threshold, a simulation of hydration methods may be initiated for determining a suitable method for hydrating, such as rehydrating, the user. The system, such as the system 100 of Fig. 1, runs different simulations of hydration methods using one or more trained ML models, for example different timings of consumption or different drink types, and a predicted effect of that simulated hydration methods for the specific user on the hydration level. Based on the output of the simulations of different hydration methods, one hydration method is selected as a recommended hydration method 304 based on a maximal effectiveness on hydration in the current context. The other simulated hydration methods may be labelled as suboptimal hydration methods 305. The recommended hydration method 304 may be communicated to the user via a user device, such as using a smartphone application. Ground truth hydration level values can be collected from a urine sensor in a toilet and/or a sanitary product, for example with every toilet visit of the user. By comparing model-based predicted hydration scores during the toilet visits to the ground truth hydration scores from that toilet visit, the one or more ML models can be adapted and retrained with new hydration level label(s).

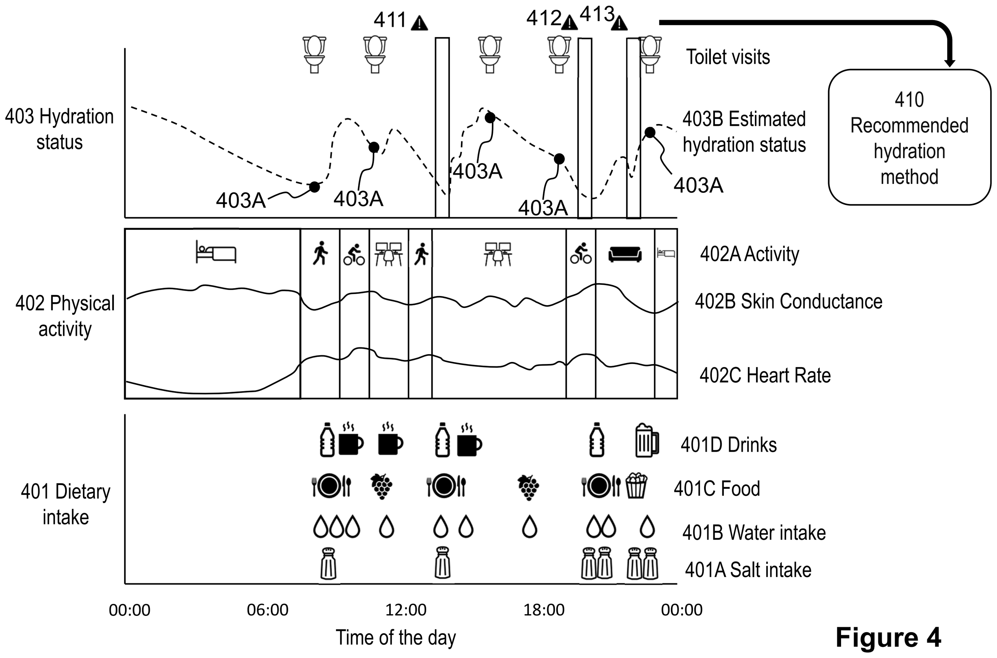

Figure 4 is a graph 400 illustrating a method for providing a recommended hydration method according to one or more examples herein. In addition to the hydration level 403 of the user, such as the USG of the user, the example further considers other hydration context data, such as a physical activity 402 and/or a dietary intake 401 of the user when simulating the hydration methods. Similar to Figure 3, the hydration level of the user may be monitored throughout the day, for example by receiving measured hydration levels 403A, such as urine hydration levels of the user, at predetermined measurement instances, for example during toilet visits of the user. Based on the measured hydration levels, a hydration status 403A may be estimated using a first and/or a second ML model. The hydration status may be an estimated current hydration level of the user at any given time and/or a predicted future hydration level of the user. In addition, further hydration context data as disclosed herein may be used as input for determining a hydration method for the user. In the example shown in Fig. 4, a dietary intake 401 may be provided as hydration context data. The dietary intake may be provided as dietary intake data, such as data 401A indicative of a salt intake of the user, data 401B indicative of a water intake of the user, data 401C indicative of a food intake of the user, and data 401D indicative of a drink intake, such as an intake of drinks other than water. The data 401C and/or 401D may be indicative of a dietary intake preference of the user. By including dietary intake preferences of the user, the recommended dietary intake for hydrating the user can be adapted based on the preferences provided. Each dietary intake data may be provided with a timestamp indicating the time of day that the dietary intake took place. The data indicative of the dietary intake may be received from the one or more input devices described herein, such as one or more sensors monitoring a dietary supply and/or via user input using the user interface.

Furthermore, the hydration context data may be indicative of a physical activity 402 of the user, such as may comprise physical activity data. The physical activity data may comprise data 402A indicative of an activity of the user, such as sleeping, walking, running, biking, working or relaxing. The physical activity data may further comprise skin conductance data 402B indicative of a physical activity of the user. The skin conductance data 402B may be indicative of an amount of sweat secreted by the user, which in turn is an indication of the physical activity of the user. The skin conductance data 402B may for example be received from a skin conductance sensor worn by the user. The physical activity data may further comprise data 402C indicative of a heart rate of the user. The heart rate may vary with the physical activity of the user and data 402C indictive of the heart rate may thus be indicative of the physical activity of the user. The physical activity data may may be provided with a timestamp indicating the time of day that the physical activity took place. In other words, besides comparing hydration method simulations based on a dietary intake of the user and providing a recommend hydration method, method may also comprise comparing different hydration method simulations based on a lifestyle, such as based on the physical activity 402 of the user and/or a dietary intake preference of the user, and predict their effect on the user's hydration levels to provide an optimized recommended hydration method for the user.

Based on the provided hydration context data, a recommended hydration method 410 may be output to a user device. The recommended hydration method may in one or more examples comprise a recommended dietary intake and a recommend time for the intake. However, the recommended hydration method may in one or more examples also indicate a recommended change in physical activity. For example, at an example time instant 411, the following recommended hydration method 410 may be output to the user: "We noticed you started walking outside while your hydration level is already on the lower end. Try to stay in the shadows and bring a drink." At a second example time instant 412, the recommended hydration method 410 may instead state: "We noticed you started cycling outside while your hydration level is already on the lower end. Get your salt concentration up soon by eating a proper meal." In a further example, such as at third example time instant 413, a third recommended hydration method may state: "We noticed you started consuming highly salty snacks, make sure to have a drink with it to keep your hydration level stable." In other words, the recommended hydration method may be tailored, such as continuously adapted, to the current state of the user.

The above embodiments are to be understood as illustrative examples of the invention. Further embodiments of the invention are envisaged. For example, [add possibilities]. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.
Examples of a system and a computer implemented method according to the disclosure are set out in the following items:
Item 1. A system comprising
   one or more processors; and
   one or more input devices for providing data,
   wherein the system is configured to:
   - receive, from a first input device of the one or more input devices, hydration context data indicative of a hydration context of a user;
   - estimate, using at least a first machine learning, ML, model and based on the hydration context data, a hydration level of the user;
   - simulate, using the first ML model, one or more hydrating methods for hydrating the user; and
   - determine, based on the one or more hydrating methods, a recommended hydration method for the user, wherein the hydration method is indicative of one or more of a type of liquid to consume and a time to consume the liquid.
Item 2. The system according to Item 1, wherein the system is configured to:
   - predict, using the first ML model trained to predict a future hydration level based on the hydration context data, a future hydration level, and
   - estimate, using a second ML model trained to estimate a current hydration level based on the hydration context data, an estimated current hydration level.
Item 3. The system according to Item 1 or 2, wherein the system is configured to:
   - input a plurality of sets of hydration context data, wherein each of the sets of hydration context data is associated with a respective hydrating method, to the first ML model, and
   - simulate a respective predicted future hydration level for each set of the plurality of sets of hydration context data.
Item 4. The system according to any one of the previous Items, wherein the system is configured to:
   - receive, from a second input device, ground truth data indicative of an actual hydration level of the user,
   - compare the obtained ground truth data with ground truth data used to train the first ML model and/or the second ML model to determine whether the obtained ground truth data comprises ground truth data values not previously used for training the first ML model and/or the second ML model,
   - determine that a number of ground truth data values not previously used for training the first and/or the second ML model is equal to or above a label threshold, and
   - retrain, using the one or more processors, one or more of the first ML model and the second ML model based on the number of ground truth data values not previously used for training.
Item 5. The system according to any one of the previous Items, wherein the first input device is a sensor or a user interface.
Item 6. A computer implemented method for providing a recommended hydration method for a user, wherein the method comprises:
   - obtaining (S202) hydration context data indicative of a hydration context of a user;
   - estimating (S204), using at least a first machine learning, ML, model and based on the hydration context data, a hydration level of the user;
   - simulating (S206), using the first ML model, one or more hydrating methods for hydrating the user; and
   - determining (S208), based on the one or more simulated hydrating methods, a recommended hydration method for the user.
Item 7. The method according to Item 6, wherein estimating (S204) comprises one or more of:
   - predicting (S204A), using the first ML model trained to predict a future hydration level based on the hydration context data, a future hydration level, and
   - estimating (S204B), using a second ML model trained to estimate a current hydration level based on the hydration context data, an estimated current hydration level.
Item 8. The method according to Item 6 or 7, wherein the step of simulating (S206) the one or more hydrating methods comprises:
   - inputting (S206A) a plurality of sets of hydration context data, wherein each of the sets of hydration context data is associated with a respective hydrating method, to the first ML model, and
   - simulating (S206B) a respective predicted future hydration level for each set of the plurality of sets of hydration context data.
Item 9. The method according to Item 8, wherein the step of determining (S208) comprises:
   - determining (S208A) at least one of the plurality of sets of hydration context data resulting in a predicted future hydration level being above the hydration threshold.
Item 10. The method according to any one of the Items 6 to 9, wherein the method comprises:
   - outputting (S210) the determined recommended hydrating method to a user device.
Item 11. The method according to any one of the Items 6 to 10, wherein the step of simulating (S206) is performed upon the estimated hydration level being equal to or below a hydration threshold.
Item 12. The method according to any one of the Items 6 to 11, wherein the hydration context data comprises data indicative of one or more of:
   - a dietary intake,
   - a physical activity of the user,
   - a geographical location of the user,
   - one or more temperatures associated with the user,
   - a dietary intake preference of the user,
   - a health status, and
   - a demographic of the user.
Item 13. The method according to Item 12, wherein the data indicative of the dietary intake comprises data indicative of one or more of:
   - a type of liquid,
   - an amount of liquid,
   - a nutrient content of the liquid,
   - a type of food,
   - an amount of food,
   - a nutrient content of the food, and
   - a time of day of the intake of the food and/or liquid,
   taken in by the user.
Item 14. The method according to any one of the Items 6 to 13, wherein the hydration method is indicative of one or more of a type of liquid to consume and a time to consume the liquid.
Item 15. The method according to any one of the Items 6 to 14, wherein the step of obtaining (S202) hydration context data comprises one or more of:
   - receiving (S202A) the hydration context data from a first sensor, and
   - receiving (S202B) the hydration context data from a user interface.
Item 16. The method according to any one of the Items 6 to 15, wherein the first ML model is trained based on hydration context data and ground truth data indicative of an actual hydration level of the user to output a future hydration level of the user, wherein the hydration context data comprises at least data indictive of a dietary intake of the user.
Item 17. The method according to any one of the Items 6 to 16, wherein the second ML model is trained based on hydration context data and ground truth data indicative of an actual hydration level of the user to output an estimated current hydration level of the user, wherein the hydration context data comprises data indictive of one or more of a dietary intake of the user and a urine hydration level of the user.
Item 18. The method according to Item 16 or 17, wherein the method comprises:
   - obtaining (S212) ground truth data indicative of an actual hydration level of the user,
   - comparing (S214) the obtained ground truth data with ground truth data used to train the first ML model and/or the second ML model to determine whether the obtained ground truth data comprises ground truth data values not previously used for training the first ML model and/or the second ML model, and
   - upon determining that a number of ground truth data values not previously used for training the first and/or the second ML model is equal to or above a label threshold, retraining (S216) one or more of the first ML model and the second ML model based on the number of ground truth data values not previously used for training.

## Claims

1. A system (100) comprising
one or more processors (102); and
one or more input devices (101) for providing data,
wherein the system (100) is configured to:
- receive, from a first input device (101A) of the one or more input devices (101), hydration context data indicative of a hydration context of a user;
- estimate, using at least a first machine learning, ML, model (106) and based on the hydration context data, a hydration level of the user;
- simulate, using the first ML model (106), one or more hydrating methods for hydrating the user; and
- determine, based on the one or more hydrating methods, a recommended hydration method for the user, wherein the hydration method is indicative of one or more of a type of liquid to consume and a time to consume the liquid.

2. The system according to claim 1, wherein the system (100) is configured to:
- predict, using the first ML model (106) trained to predict a future hydration level based on the hydration context data, a future hydration level, and
- estimate, using a second ML model (107) trained to estimate a current hydration level based on the hydration context data, an estimated current hydration level.

3. The system according to claim 1 or 2, wherein the system (100) is configured to:
- input a plurality of sets of hydration context data, wherein each of the sets of hydration context data is associated with a respective hydrating method, to the first ML model (106), and
- simulate a respective predicted future hydration level for each set of the plurality of sets of hydration context data.

4. The system according to any one of the previous claims, wherein the system (100) is configured to:
- receive, from a second input device (101B), ground truth data indicative of an actual hydration level of the user,
- compare the obtained ground truth data with ground truth data used to train the first ML model (106) and/or the second ML model (107) to determine whether the obtained ground truth data comprises ground truth data values not previously used for training the first ML model and/or the second ML model,
- determine that a number of ground truth data values not previously used for training the first and/or the second ML model is equal to or above a label threshold, and
- retrain, using the one or more processors (102), one or more of the first ML model and the second ML model based on the number of ground truth data values not previously used for training.

5. The system according to any one of the previous claims, wherein the first input device (101A) is a sensor or a user interface.

6. A computer implemented method for providing a recommended hydration method for a user, wherein the method comprises:
- obtaining (S202) hydration context data indicative of a hydration context of a user;
- estimating (S204), using at least a first machine learning, ML, model and based on the hydration context data, a hydration level of the user;
- simulating (S206), using the first ML model, one or more hydrating methods for hydrating the user; and
- determining (S208), based on the one or more simulated hydrating methods, a recommended hydration method for the user.

7. The method according to claim 6, wherein estimating (S204) comprises one or more of:
- predicting (S204A), using the first ML model trained to predict a future hydration level based on the hydration context data, a future hydration level, and
- estimating (S204B), using a second ML model trained to estimate a current hydration level based on the hydration context data, an estimated current hydration level.

8. The method according to claim 6 or 7, wherein the step of simulating (S206) the one or more hydrating methods comprises:
- inputting (S206A) a plurality of sets of hydration context data, wherein each of the sets of hydration context data is associated with a respective hydrating method, to the first ML model, and
- simulating (S206B) a respective predicted future hydration level for each set of the plurality of sets of hydration context data.

9. The method according to claim 8, wherein the step of determining (S208) comprises:
- determining (S208A) at least one of the plurality of sets of hydration context data resulting in a predicted future hydration level being above the hydration threshold.

10. The method according to any one of the claims 6 to 9, wherein the method comprises:
- outputting (S210) the determined recommended hydrating method to a user device.

11. The method according to any one of the claims 6 to 10, wherein the step of simulating (S206) is performed upon the estimated hydration level being equal to or below a hydration threshold.

12. The method according to any one of the claims 6 to 11, wherein the hydration context data comprises data indicative of one or more of:
- a dietary intake,
- a physical activity of the user,
- a geographical location of the user,
- one or more temperatures associated with the user,
- a dietary intake preference of the user,
- a health status, and
- a demographic of the user.

13. The method according to any one of the claims 6 to 12, wherein the hydration method is indicative of one or more of a type of liquid to consume and a time to consume the liquid.

14. The method according to any one of the claims 6 to 13, wherein the step of obtaining (S202) hydration context data comprises one or more of:
- receiving (S202A) the hydration context data from a first sensor, and
- receiving (S202B) the hydration context data from a user interface.

15. The method according to any one of the claims 6 to 14, wherein the first ML model is trained based on hydration context data and ground truth data indicative of an actual hydration level of the user to output a future hydration level of the user, wherein the hydration context data comprises at least data indictive of a dietary intake of the user.

16. The method according to any one of the claims 6 to 15, wherein the second ML model is trained based on hydration context data and ground truth data indicative of an actual hydration level of the user to output an estimated current hydration level of the user, wherein the hydration context data comprises data indictive of one or more of a dietary intake of the user and a urine hydration level of the user.
